(19) Europäisches Patentamt
European Patent Office
Office européen des brevets

(11) Veröffentlichungsnummer: **0 396 215 A1**

# (12) EUROPÄISCHE PATENTANMELDUNG

(21) Anmeldenummer: **90250106.3**

(22) Anmeldetag: **27.04.90**

(51) Int. Cl.5: **C07D 261/12, C07D 413/04, A01N 43/80**

(30) Priorität: **02.05.89 DE 3914969**

(43) Veröffentlichungstag der Anmeldung:
**07.11.90 Patentblatt 90/45**

(84) Benannte Vertragsstaaten:
**AT BE CH DE DK ES FR GB GR IT LI LU NL SE**

(71) Anmelder: **SCHERING AKTIENGESELLSCHAFT**
**Müllerstrasse 170/178**
**D-1000 Berlin 65(DE)**

(72) Erfinder: **Hübl, Dieter, Dr.**
**Käthe-Kollwtz-Strasse 123**
**D-4712 Werne(DE)**
Erfinder: **Pieroh, Ernst**
**Holme 25**
**D-2263 Risum-Lindholm(DE)**

## (54) 5-Substituierte 3-Arylisoxazol-Derivate, deren Herstellung und Verwendung als Schädlingsbekämpfungsmittel.

(57) Es werden neue 5-Substituierte 3-Arylisoxazol-Derivate der allgemeinen Formel I

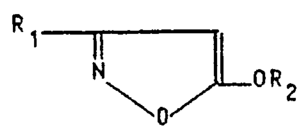

(I),

worin $R^1$ und $R^2$ die in der Beschreibung angegebene Bedeutung haben, Verfahren zu ihrer Herstellung und ihre Verwendung als Schädlingsbekämpfungsmittel, insbesondere gegen Nematoden beschrieben.

EP 0 396 215 A1

## 5-Substituierte 3-Arylisoxazol-Derivate, deren Herstellung und Verwendung als Schädlingsbekämpfungsmittel

Die Erfindung betrifft neue 5-substituierte 3-Arylisoxazol-Derivate, deren Herstellung und ihre Verwendung als Schädlingsbekämpfungsmittel, insbesondere gegen Nematoden.

Arylisoxazole mit nematizider Wirkung sind bereits bekannt, wie z.B. aus U.S.P. 3,781,438.

Die bekannten Verbindungen haben jedoch den Nachteil, daß sie entweder nicht ausreichend pflanzenverträglich oder aber nicht ausreichend wirksam sind.

Die Aufgabe der vorliegenden Erfindung war es, Verbindungen bereitzustellen, die insbesondere gegen Nematoden gut wirksam sind, ohne gleichzeitig pflanzenunverträglich zu sein.

Es wurde nun gefunden, daß 5-substituierte 3-Arylisoxazol-Derivate der allgemeinen Formel I

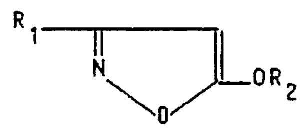

(I),

worin

$R^1$ einen ein- oder mehrfach, gleich oder verschieden durch Halogen, $C_{1-14}$-Alkyl, $C_{3-6}$-Cycloalkyl, $C_{1-4}$-Alkoxy, $C_{1-4}$-Alkylthio, Di-$C_{1-4}$alkylamino, $C_{1-6}$-Alkoxycarbonyl, Halogen-$C_{1-4}$-alkyl, Halogen-$C_{1-4}$-alkoxy, Halogen-$C_{1-4}$-alkylthio, Halogen-$C_{1-6}$-alkoxycarbonyl, Halogen-$C_{3-6}$-cycloalkyl, Halogen-$C_{3-6}$-cycloalkylmethoxy, Halogen-$C_{3-6}$-cycloalkylmethylthio, Nitro, Cyano, Amino, Phenoxy, Halogenphenoxy, Phenylthio oder Halogenphenylthio substituierten Phenyl-, Biphenyl-, Naphthyl-, Furyl-, Thienyl- oder Pyridylrest und

$R^2$ einen ein- oder mehrfach gleich oder verschieden durch Halogen substituierten $C_{1-12}$-Alkyl-, $C_{2-12}$-Alkenyl-, $C_{2-12}$-Alkinyl-, $C_{3-6}$-Cycloalkyl- oder $C_{3-6}$-Cycloalkylmethylrest bedeutet, eine überraschend gute nematizide Wirkung bei gleichzeitig guter Pflanzenverträglichkeit aufweisen.

Die erfindungsgemäßen Verbindungen zeigen darüberhinaus aber auch eine gute Wirkung gegen beißende und saugende Insekten sowie gegen Insekteneier und gegen Milben.

Die Bezeichnung "Halogenalkyl" besagt, daß ein oder mehrere Wasserstoffatome des Alkylrestes durch Halogen ersetzt sind. Unter Halogen ist Cl, F, Br und Jod zu verstehen.

Die erfindungsgemäßen Verbindungen der Formel I lassen sich nach an sich bekannten Methoden dadurch herstellen, daß man Verbindungen der Formel II

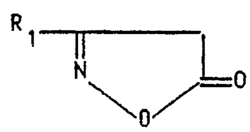

(II),

wobei $R_1$ die in Formel I angegebene Bedeutung hat, mit Verbindungen der Formel Z-$R_2$, wobei Z eine Fluchtgruppe wie z.B. Halogen, Mesylat und Tosylat ist und $R_2$ die in Formel I angegebene Bedeutung hat, in einem inerten Lösungsmittel oder Lösungsmittelgemisch bei gebenenfalls erhöhter Temperatur und gegebenenfalls bei erhöhtem Druck in Gegenwart einer Base umsetzt.

Als Rasen können organische und anorganische Basen eingesetzt werden, wie z.B. tertiäre Amine wie Triethylamin und Tripropylamin, Alkali- und Erdalkalimetall-hydride, -hydroxide, -carbonate und -bicarbonate, aber auch Alkalialkoholate, wie z.B. Natriummethylat oder Kalium-tert.-butylat.

Für die Herstellung der erfindungsgemäßen Verbindungen geeignete Lösungsmittel sind z.B. Diethylether, Dioxan und Tetrahydrofuran, aliphatische und aromatische Kohlenwasserstoffe wie Toluol und Petrolether; halogenierte Kohlenwasserstoffe wie Chlorbenzol, Methylenchlorid, Tetrachlorkohlenstoff, Chloroform; Nitrile wie Acetonitril, Propionitril; N,N-dialkylierte Amide wie Dimethylformamid; Ketone wie Aceton, Methylethylketon; Dimethylsulfoxid, Sulfolan sowie Wasser und Alkohole, z.B. Methanol, Ethanol, Isopropanol oder Butanol und die Gemische solcher Lösungsmittel untereinander.

Die Reaktion wird in Abhängigkeit von den Reaktanden bei Temperaturen zwischen -70 °C bis 120 °C durchgeführt. Auch in Abhängigkeit von den Reaktanden kann die Anwendung von Druck zweckmäßig sein. Der dann angewendete Druck liegt im Bereich zwischen 1 bis 25 bar. Die Reaktionsdauer beträgt ca. 0,5 bis 48 Stunden. Das Reaktionsgemisch wird anschließend auf Eis/Wasser gegossen, extrahiert und in an

sich bekannter Weise aufgearbeitet. Die anfallenden Produkte lassen sich in üblicher Weise durch Umkristallisation, Vakuumdestillation oder Säulenchromatographie reinigen.

Die als Ausgangsmaterialien verwendeten Verbindungen der Formel II sind teilweise bekannt oder lassen sich nach an sich bekannten Verfahren herstellen (z.B. USP 3,781,438; Chem.Ber. 110, 2922-2938 (1977) und J.Chem.Soc. C 1971, 1945).

Aufgrund der nematiziden Wirksamkeit bei guter Pflanzenverträglichkeit können die erfindungsgemäßen Verbindungen mit Erfolg im Pflanzenschutz als Schädlingsbekämpfungsmittel in der Landwirtschaft, im Wein-, Obst- und Gartenbau und in Forstkulturen eingesetzt werden.

Zu den pflanzenparasitären Nematoden, die erfindungsgemäß bekämpft werden können, gehören beispielsweise die Wurzelgallen-Nematoden, wie Meloidogyne incognita, Meloidogyne hapla, Meloidogyne javanica, die Zysten bildenden Nematoden, wie Globodera rostochiensis, Heterodera schachtii, Heterodera avenae, Heterodera glycines, Heterodera trifolii, Stock- und Blattälchen, wie Ditylenchus dipsaci, Ditylenchus destructor, Aphelenchoides ritzemabosi, Pratylenchus neglectus, Pratylenchus penetrans, Pratylenchus curvitatus sowie Tylenchorhynchus dubius, Tylenchorhynchus claytoni, Rotylenchus robustus, Heliocotylenchus multicinctus, Radopholus similis, Belonolaimus longicaudatus, Longidorus elongatus und Trichodorus primitivus.

Aufgrund der insektiziden und akariziden Eigenschaften der erfindungsgemäßen Verbindungen bieten sie darüberhinaus Anwendungsmöglichkeiten sowohl in der Behandlung gegen Schädlinge in den verschiedensten Stadien der Kulturpflanzen als auch gegen Schädlinge an Mensch und Tier.

Die Anwendung der erfindungsgemäßen Wirkstoffe erfolgt in Form ihrer handelsüblichen Formulierungen und/oder aus diesen Formulierungen bereiteten Anwendungsformen.

Der Wirkstoffgehalt der aus den handelsüblichen Formulierungen bereiteten Anwendungsformen kann in weiten Bereichen variieren. Die Aufwandmenge für die Bekämpfung von Nematoden pro Hektar beträgt etwa 0,03 kg bis etwa 10 kg, vorzugsweise etwa 0,3 kg bis etwa 6 kg.

Die Wirkstoffe oder deren Mischungen können in die üblichen Formulierungen überführt werden wie Lösungen, Emulsionen, Spritzpulver, Suspensionen, Pulver, Stäubemittel, Schäume, Pasten, lösliche Pulver, Granulate, Aerosole, Suspensions-Emulsionskonzentrate, Saatgutpuder, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä. sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden.

Als flüssige Lösungsmittel kommen im wesentlichen infrage aromatische Kohlenwasserstoffe wie Xylol, Toluol oder Alkylnaphthaline, chlorierte Aromaten oder chlorierte aliphatische Kohlenwasserstoffe wie Chlorbenzole, Chlorethylene oder Methylenchlorid, aliphatische Kohlenwasserstoffe wie Cyclohexan oder Paraffine, z.B. Erdölfraktionen, Alkohole wie Butanol, Glycol sowie deren Ether und Ester, Ketone wie Aceton, Methylethylketon, Methylisobutylketon oder Cyclohexanon, stark polare Lösungsmittel wie Dimethylformamid und Dimethylsulfoxid sowie Wasser.

Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase wie Halogenkohlenwasserstoffe sowie Butan, Propan, Stickstoff und Kohlendioxid.

Als feste Trägerstoffe kommen infrage natürliche Gesteinsmehle wie Kaoline, Tonerden, Talkum, Kreide, Quarz, Attapulgit, Montmorillonit oder Diatomeenerde und synthetische Gesteinsmehle wie hochdisperse Kieselsäure, Aluminiumoxid und Silikate sowie als feste Trägerstoffe für Granulate gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel.

Als Emulgatoren bzw. schaumerzeugende Mittel seien genannt nicht-ionogene und anionische Emulgatoren wie Polyoxyethylen-Fettsäure-Ester, Polyoxyethylen-Fettalkohol-Ether, z.B. Alkylaryl-polyglykolether, Alkylsulfonate, Arylsulfonate sowie Eiweißhydrolysate.

Als Dispergiermittel seien z.B. Lignin, Sulfitablaugen und Methylcellulose genannt.

Es können in den Formulierungen auch Haftmittel wie Carboxymethylcellulose, natürliche und synthetische pulvrige, körnige oder latexförmige Polymere verwendet werden wie Gummi arabicum, Polyvinylalkohol und Polyvinylacetat.

Weiterhin können Farbstoffe wie anorganische Pigmente, z.B. Eisendioxid, Titanoxid, Ferrocyanblau und organische Farbstoffe wie Alizarin-, Azo-Metallphthalocyaninfarbstoffe und Spurennährstoffe wie Salze von Eisen, Mangan, Bor, Kupfer, Kobalt, Molybdän und Zink mitverwendet werden.

Die Formulierungen enthalten im allgemeinen zwischen 0,1 und 95 Gewichtsprozent Wirkstoff, vorzugsweise zwischen 0,5 und 90 %.

Beispiele für Formulierungen sind:

I. **Spritzpulver**

10 Gewichtsteile der Verbindung gemäß Beispiel 1 werden mit 12 Gewichtsteilen Calciumsalz der Ligninsulfonsäure, 76 Gewichtsteilen Kaolin und 2 Gewichtsteilen Dialkylnaphthalinsulfonat innig vermischt und vermahlen.

II. **Stäubepulver**

2,5 Gewichtsteile der Verbindung gemäß Beispiel 1 werden in 10 Teilen Methylenchlorid gelöst und auf eine Mischung von 25 Gewichtsteilen pulverförmige Kieselsäure und 71,5 Gewichtsteilen Talkum sowie 1 Gewichtsteil Sudanrot gegeben. Das Lösungsmittel wird im Vakuum entfernt und der Rückstand fein vermahlen.

III. **Granulat**

5 Gewichtsteile der Verbindung gemäß Beispiel 1 werden in 10 Teilen Methylenchlorid gelöst und auf 95 Gewichtsteile granuliertes Attapulgit der Korngröße 0,3 - 0,8 mm aufgesprüht und getrocknet.

IV. **Emulsionskonzentrat**

20 Gewichtsteile der Verbindung gemäß Beispiel 1 werden in einer Mischung aus 75 Gewichtsteilen Isophoron und 5 Gewichtsteilen eines Gemisches aus 30 Teilen Phenylsulfonat-Calciumsalz, 30 Teilen Rhizinus-Polyglycolat mit 40 Mol% Ethylenoxid und 40 Teilen eines Copolymeren von Propylen- und Ethylenoxid gelöst.

Die folgenden Beispiele beschreiben die Herstellung der erfindungsgemäßen Verbindungen.

**Herstellungsbeispiel 1**

3-(4-Chlorphenyl)-5-difluormethoxy-isoxazol

In einem 100 ml Autoklaven werden 5,87 g (0,03 mol) 3-(4-Chlorphenyl)-5-(4H)-isoxazolon in 30 ml Dioxan gelöst und mit 10 ml einer 30 %igen Kaliumhydroxidlösung versetzt. Man preßt 10 bar Chlordifluormethan hinein und erwärmt die Reaktionsmischung während 15 Stunden auf 50-60 °C. Nach Abkühlung und Entspannung des Drucks gießt man die Lösung auf 300 ml Eis/Wasser und extrahiert 3mal mit 70 ml Essigester. Die vereinigten organischen Phasen werden über Magnesiumsulfat getrocknet und eingeengt. Die Reinigung erfolgt durch Säulenchromatographie.
Ausbeute: 1,20 g ≙ 16,3 % der Theorie
Fp.: 52 °C

**Herstellungsbeispiel 2**

5-Bromdifluormethoxy-3-(4-Chlorphenyl)-isoxazol

1,5 g (0,05 mol) einer 80 %igen Natriumhydridsuspension in Paraffinöl wird einmal mit 10 ml Toluol gewaschen und in 50 ml Dimethylformamid suspendiert. Bei einer Temperatur von 10-20 °C wird eine Lösung von 5,0 g (0,026 mol) 3-(4-Chlorphenyl-5(4H)-isoxazolon in 40 ml Dimethylformamid zugetropft. Es wird 1 Stunde nachgerührt. Danach werden 10,75 g (0,052 mol) Dibromdifluormethan eingetropft und die Reaktionslösung zwei Stunden bei Raumtemperatur nachgerührt. Das Reaktionsgemisch wird auf 300 ml Eis/Wasser gegossen und dreimal mit 100 ml Essigester extrahiert. Die vereinigten organischen Phasen werden über Magnesiumsulfat getrocknet und eingeengt. Die Reinigung erfolgt durch Säulenchromatographie (Eluent: Hexan/Diethylether 9:1).
Ausbeute: 1 ,2 g ≙ 14 % der Theorie
$n_D^{20}$ : 1,54550

In analoger Weise werden die folgenden Verbindungen hergestellt:

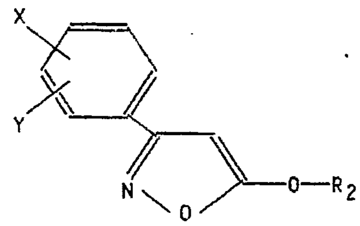

| Beispiel Nr. | X | Y | $R_2$ | Fp °C / $n_D^{20}$ |
|---|---|---|---|---|
| 3 | 4-Cl | H | $CF_2=CF(CH_2)_2$ | 63 |
| 4 | 4-Cl | H | 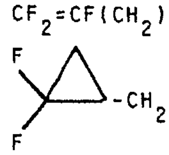 | 85 |
| 5 | 4-Cl | H | $CF_3-(CH_2)_3$ | 80 |
| 6 | 4-Cl | H | $FCH_2CH_2$ | 91 |
| 7 | H | H | $CHF_2$ | 43-45 |
| 8 | H | H | $CF_2=CF-(CH_2)_2$ | 83 |
| 9 | H | H | $CF_2Br$ | 1,5161 |
| 10 | H | H | 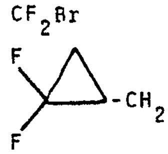 | 90-02 |
| 11 | 3-Cl | H | $CF_2Br$ | 1,5387 |
| 12 | 3-Cl | H | $CF_2=CF-(CH_2)_2$ | 52-54 |
| 13 | 2-Cl | H | $CF_2=CF(CH_2)_2$ | 1,5216 |
| 14 | 3-Cl | H | $CF_2$ | 1,5187 |
| 15 | 2-Cl | H | $CF_2$ | 1,5183 |
| 16 | 2-Cl | H | $CF_2Br$ | 1,5286 |
| 17 | $4-OCH_3$ | H | $CF_2=CF(CH_2)_2$ | 66-69 |
| 18 | $4-OCH_3$ | H | $CF_2Br$ | 1,5413 |
| 19 | H | H | $CH_2=CCl-CH_2$ | 58-60 |
| 20 | H | H | $ClCH=CH-CH_2$ | 28-30 |
| 21 | 4-Cl | 2-Cl | $CF_2=CF(CH_2)_2$ | 1,5374 |
| 22 | 4-Cl | 2-Cl | $CF_2Br$ | 1,5339 |
| 23 | 4-F | H | $CF_2Br$ | 1,4797 |

| Beispiel Nr. | X | Y | $R_2$ | Fp °C / $n_D^{20}$ | |
|---|---|---|---|---|---|
| 24 | 4-F | H | $CF_2=CF(CH_2)_2$ | 58-59 | |
| 25 | 4-Cl | 2-Cl | $CF_2$ | | 1,5418 |
| 26 | 4-F | H | $CF_2$ | | 1,5016 |
| 27 | 4-$CH_3$ | H | $CF_2=CF(CH_2)_2$ | 84-86 | |
| 28 | 4-$CH_3$ | H | $CF_2$ | 49-51 | |
| 29 | 4-$CH_3$ | H | $CF_2Br$ | | 1,5293 |
| 30 | 3-F | H | $-CH_2CH_2-CHF=CF_2$ | 63-65 | |
| 31 | 3-F | H | $-CF_2Br$ | | 1,5136 |
| 32 | 3-F | H | $-CHF_2$ | | 1,5023 |
| 33 | 4-$CF_3$ | H | $-CHF_2$ | 40-41 | |
| 34 | 4-$CF_3$ | H | $-CH_2-CH_2-CHF=CF_2$ | 60-62 | |
| 35 | 3-Cl | 4-Cl | $-CH_2-CH_2-CHF=CF_2$ | 39-41 | |
| 36 | 3-Cl | 4-Cl | $-CHF_2$ | 47-48 | |
| 37 | 2-F | 6-F | $-CH_2-CH_2-CHF=CF_2$ | 61-63 | |
| 38 | 2-F | 6-F | $-CHF_2$ | 42-45 | |
| 39 | 4-$CF_3$ | H | $-CF_2Br$ | | 1,48518 |
| 40 | 3-Cl | 4-Cl | $-CF_2Br$ | | 1,56158 |
| 41 | 2-F | H | $-CHF_2$ | | 1,50352 |
| 42 | 2-F | H | $-CF_2Br$ | | 1,51036 |
| 43 | 2-F | H | $-CH_2-CH_2-CHF=CF_2$ | 66-68 | |

Das nachfolgende Beispiel zeigt die Anwendungsmöglichkeit der erfindungsgemäßen Verbindungen.

## Anwendungsbeispiel A

### Bekämpfung von Wurzelgallennematoden, Meloidogyne incognita

5 %ige pulverförmige Wirkstoffzubereitungen werden gleichmäßig mit Boden vermischt, der mit den Testnematoden stark verseucht ist. Danach füllt man den behandelten Boden in 0,5 Liter fassende Tonschalen, sät Gurkensamen ein und kultiviert bei einer Bodentemperatur von 25-27 °C im Gewächshaus. Nach einer Kulturdauer von 25-28 Tagen werden die Gurkenwurzeln ausgewaschen, im Wasserbad auf Nematodenbefall (Wurzelgallen) untersucht und der Wirkungsgrad des Wirkstoffes im Vergleich zur verseuchten Kontrolle in % bestimmt. Wird der Nematodenbefall vollständig vermieden, wird der Wirkungsgrad 100 % gesetzt.

Bei einer Dosis von 25 mg aktiver Substanz je Liter Boden konnte ein Nematodenbefall durch Meloidogyne incognita durch die Verbindungen der Beispiele 2, 3, 8, 9, 10, 12, 13, 16, 17, 18, 21, 23 und 24 vollständig (90-100 %ig) vermieden werden.

## Anwendungsbeispiel B

**Wirkung der prophylaktiscnen Futterbehandlung gegen die Braune Reiszikade (Nilaparvata lugens Stal)**

Reissämlinge (Oryza sativa L.) im Zweiblattstadium (etwa 10 je Polystyroltopf von 6,5 x 6,5 cm) werden unbehandelt bzw. nach Tauchbehandlung mit 0,1 % Wirkstoff enthaltender wäßriger Zubereitung bis zum Antrocknen der Flüssigkeit im Labor aufgestellt. Dann wird über jeden Topf ein Polystyrolzylinder gestülpt, durch dessen obere Öffnung etwa 30 mit Kohlendioxid betäubte Individuen Nilaparvata lugens im 4.- 5. Stadium eingebracht werden. Nach Verschließen der Öffnung mit einem engmaschigen Sieb werden die Töpfe zwei Tage lang bei 28°C und 16 h/Tag Licht gehalten. Dann wird die prozentuale Mortalität bestimmt und unter Bezug auf die unbehandelte Kontrolle die Wirkung nach Abbott berechnet.

Die erfindungsgemäße Verbindung gemäß Beispiel Nr. 6 zeigte eine Wirkung von 80 % oder mehr.

**Anwendungsbeispiel C**

**Wirkung der prophylaktischen Futterbehandlung gegen die Gemeine Bohnenspinnmilbe (Tetranychus urticae Koch)**

Aus entwickelten Primärblättern der Buschbohne (Phaseolus vulgaris nanus Aschers.) werden runde Blattscheiben mit einem Durchmesser von 14 mm gestanzt und unbehandelt bzw. nach Tauchbehandlung mit einer 0,1 % Wirkstoff enthaltenden wäßrigen Zubereitung auf nasses Filterpapier gelegt, wobei die Blattunterseite nach oben gerichtet ist. Nach Antrocknen der so behandelten Proben werden sechs erwachsene Weibchen von Tetranychus urticae auf jede Blattscheiben gesetzt und für 3 Tage bei 25°C und 16 h pro Tag bei Licht gehalten (vier Wiederholungen). Dann werden die toten und lebenden Weibchen gezählt und entnommen. Gleichfalls werden die abgelegten Eier gezählt. Nach weiteren sieben Tagen werden die lebenden Larven gezählt, und unter Bezug auf die unbehandelte Kontrolle wird nach Abbott die Gesamtwirkung berechnet, die sich aus der Wirkung gegen Adulte und gegen Eier zusammensetzt.

Die erfindungsgemäßen Verbindungen gemäß den Beispielen 6 und 18 zeigten eine 80 - 100 %ige Gesamtwirkung gegen Tetranychus urticae.

**Anwendungsbeispiel D**

**Abtötende Wirkung auf Eier/Larven des Maiswurzelwurmes (Diabrotica undeccimpunctata)**

Die erfindungsgemäßen Verbindungen werden als wäßrige Zubereitung mit einer Wirkstoffkonzentration von 0,1 % eingesetzt. Von diesen Wirkstoffzubereitungen werden 0,2 ml auf den Boden einer Polystyrolpetrischale sowie auf den darin enthaltenden Maiskeimling und auf die Schalenzentrum befindlichen ca. 50 Eier des Maiswurzelwurmes (Diabrotica undecimpunctata) pipettiert. Die verschlossenen Schalen werden bis zu 7 Tagen bei 25°C unter Langtagbedingungen aufgestellt. Kriterium für die Wirkungsbeurteilung ist die Abtötung von Eiern oder der frisch schlüpfenden Larven bei Versuchsende.

Die erfindungsgemäßen Verbindungen gemäß den Beispielen 23, 26, 28 und 29 zeigten eine 80 - 100 %ige Wirkung.

**Anwendungsbeispiel E**

**Ovizide Wirkung auf Eiablagen der Baumwelleule (Meliothis virescens)**

Die erfindungsgemäßen Verbindungen werden als wäßrige Zubereitung mit einer Wirkstoffkonzentration von 0,1 % eingesetzt. In diese Wirkstoffzubereitungen werden einen Tag alte Eiablagen, die von befruchteten Falterweibchen auf Filterpapier abgesetzt worden waren, bis zur völligen Benetzung getaucht und für vier Tage bei 25°C unter Langtagbedingungen in geschlossenen Petrischalen deponiert. Kriterium für die Wirkungsbeurteilung ist die prozentuale Schlupfverhinderung im Vergleich zu unbehandelten Eiablagen.

Die erfindungsgemäßen Verbindungen gemäß den Beispielen 6, 26, 28 und 29 zeigten eine 80 - 100

%ige Wirkung.

## Ansprüche

1. 5-Substituierte 3-Arylisoxazol-Derivate der allgemeinen Formel I

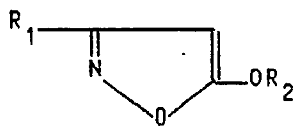

(I),

worin
$R^1$ einen ein- oder mehrfach, gleich oder verscheiden durch Halogen, $C_{1-14}$-Alkyl, $C_{3-6}$-Cycloalkyl , $C_{1-4}$-Alkoxy, $C_{1-4}$-Alkylthio, Di-$C_{1-4}$-alkylamino, $C_{1-6}$-Alkoxycarbonyl, Halogen-$C_{1-4}$-alkyl, Halogen-$C_{1-4}$-alkoxy, Halogen-$C_{1-4}$-alkylthio, Halogen-$C_{1-6}$-alkoxycarbonyl, Halogen-$C_{3-6}$-cyclo-alkyl, Halogen-$C_{3-6}$-cycloalkylmethoxy, Halogen-$C_{3-6}$-cycloalkylmethylthio, Nitro, Cyano, Amino, Phenoxy, Halogenphenoxy, Phenylthio oder Halogenphenylthio substituierten Phenyl-, Biphenyl-, Naphthyl-, Furyl-, Thienyl- oder Pyridylrest und
$R^2$ einen ein- oder mehrfach gleich oder verschieden durch Halogen substituierten $C_{1-12}$-Alkyl-, $C_{2-12}$-Alkenyl-, $C_{2-12}$-Alkinyl-, $C_{3-6}$-Cycloalkyl- oder $C_{3-6}$-Cycloalkylmethylrest bedeutet.

2. Verfahren zur Herstellung von Verbindungen der allgemeinen Formel I, dadurch gekennzeichnet, daß man Verbindungen der Formel II

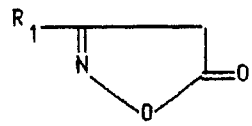

(II),

wobei $R_1$ die in Formel I angegebene Bedeutung hat, mit Verbindungen der Formel Z-$R_2$, wobei Z eine Fluchtgruppe wie z.B. Halogen, Mesylat und Tosylat ist und $R_2$ die in Formel I angegebene Bedeutung hat, in einem inerten Lösungsmittel oder Lösungsmittelgemisch bei gebenenfalls erhöhter Temperatur und gegebenenfalls bei erhöhtem Druck in Gegenwart einer Base umsetzt.

3. Verwendung von Verbindungen gemäß Anspruch 1 der Formel I zur Bekämpfung von Schädlingen, insbesondere Nematoden.

4. Schädlingsbekämpfungsmittel gekennzeichnet durch einen Gehalt an mindestens einer Verbindung gemäß Anspruch 1 der Formel I.

5. Schädlingsbekämpfungsmittel gemäß Anspruch 4 in Mischung mit Träger-und/oder Hilfsstoffen.

Europäisches Patentamt

# EUROPÄISCHER RECHERCHENBERICHT

Nummer der Anmeldung

EP 90250106.3

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl.⁵) |
|---|---|---|---|
| A | US - A - 3 879 533 (CARR) * Patentanspruch 1 * -- | 1,3-5 | C 07 D 261/12<br>C 07 D 413/04<br>A 01 N 43/80 |
| A | US - A - 3 879 532 (HASS) * Patentanspruch 1 * -- | 1,3-5 | |
| A | CHEMICAL ABSTRACTS, Band 109, Nr. 9, 29. August 1988 Columbus, Ohio, USA CHIARINO D. et al. "Synthesis of new isoxazole amino alcohols" Seite 686, Spalte 2, Zusammenfassung-Nr. 73 369f & J. Heterocycl. Chem. 1988, 25(1), 337-42 -- | 1 | |
| A | CHEMICAL ABSTRACTS, Band 89, Nr. 25, 18. Dezember 1978 Columbus, Ohio, USA KOMENDANTOV, M.I. et al. "Thermal isomerization of 5-methoxy-3-arylisoxazoles to methyl 3-aryl-2H-azirine-2-carboxylates" Seite 531, Spalte 2, Zusammenfassung-Nr. 214 685p & Khim. Geterotsikl. Soedin 1978 (8), 1053-6 -- | 1 | RECHERCHIERTE SACHGEBIETE (Int Cl.⁵)<br>C 07 D 261/00<br>C 07 D 413/00 |
| A | CHEMICAL ABSTRACTS, Band 71, Nr. 11, 15. September 1969 Columbus, Ohio, USA NISHIWAKI, TAROZAEMON "Heterocyclic chemistry. III. Thermally induced skeletal rearrangement of 5-alkoxyisoxazoles into alkyl 2H-azirine- | 1 | |

Der vorliegende Recherchenbericht wurde für alle Patentansprüche erstellt.

| Recherchenort | Abschlußdatum der Recherche | Prüfer |
|---|---|---|
| WIEN | 25-06-1990 | HAMMER |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

Europäisches Patentamt

**EUROPÄISCHER RECHERCHENBERICHT**

Nummer der Anmeldung

-2-

EP 90250106.3

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl 5) |
|---|---|---|---|
| | -2-carboxylates"<br>Seite 382, Spalte 1, Zusammen-<br>fassung-Nr. 49 823a<br>& Tetrahedron Lett. 1969, (25), 2049-52<br>---- | | |
| | | | RECHERCHIERTE SACHGEBIETE (Int Cl 5) |

Der vorliegende Recherchenbericht wurde für alle Patentansprüche erstellt.

| Recherchenort | Abschlußdatum der Recherche | Prüfer |
|---|---|---|
| WIEN | 25-06-1990 | HAMMER |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument